# EUROPEAN PATENT APPLICATION

(11) **EP 2 322 188 A1**
(43) Date of publication of application: **18.05.2011**
(21) Application number: 09174318.7
(22) Date of filing: 28.10.2009
(51) Int. Cl.: A61K 31/714, A61P 17/00, A61K 9/00

(54) **Treatment of Inflammatory and Hyperproliferative Skin disease**

(71) Applicant: Melnik, Bodo, 33330 Gütersloh (DE)
(72) Inventor: Melnik, Bodo, 33330 Gütersloh (DE)
(74) Representative: von Kreisler Selting Werner

(57) **Abstract**

Use of nitrosyl-cobalamin for the preparation of a medicament for the treatment of a inflammatory and hyperproliferative skin disease in human and veterinary medicine selected from the group consisting of eczema, atopic dermatitis, psoriasis, acne and rosacea.

## Description

The present invention relates to the treatment of inflammatory and hyperproliferative skin diseases in human medicine and veterinary medicine.

Acne is a chronic inflammatory disease of the pilosebaceous unit, mostly affecting the sebaceous follicles. Major contributors of acne pathogenesis are abnormal follicular differentiation with increased cornification, enhanced sebaceous gland activity with hyperseborrhea, bacterial hypercolonization, inflammation as wells as immunological host reactions. Sebaceous glands function continuously in excreting sebum to the skin surface with an average sebaceous cell transition time of 14 days. Androgens play an essential role for the stimulation of the size of sebocytes and sebum production as well as keratinocyte proliferation in the ductus seboglandularis and the acroinfundibulum. Exogeneous androgen excess or hyperandrogenism are associated with increased sebum production and the development of severe acne. Acne-prone skin exhibits a higher androgen receptor density and higher 5α-reductase type-I activity than not involved skin. Anti-androgens reduce the synthesis of sebaceous lipids and improve acne, whereas androgen-insensitive subjects who lack functional androgen receptors do not produce sebum and do not develop acne. The development of inflammatory papules, pustules and nodules is a common problem of acne leading to moderate to severe inflammation of sebaceous gland-rich skin regions and subsequent scar formation.

Rosacea is an inflammatory skin disease appearing in areas with high density of sebaceous glands. The primary clinical features of rosacea include flushing, inflammatory papules, pustules, and teleangiectases. In rosacea mesenchymal tissue has an increased tendency of proliferation: sebaceous glands with sebaceous hyperplasia, connective tissue with fibrosis and formation of rhinophyma, and increased angiogenesis with formation of multiple teleangiectases. Disturbed homeostasis of vascular and fibroblast growth factors have been implicated in the pathogenesis of rosacea. Roscacea of grade II and III is often associated with skin infiltrates of activated neutrophils and other inflammatory cells. Hyperproliferation of vascular cells, sebaceous glands and connective tissue are especially associated with rosacea III.

Psoriasis is one of the most common chronic inflammatory skin disorders, affecting about 2% of the general population. Prevalence rates in Europe are quoted to be about 1·5%, whereas in the U.S.A. the prevalence is estimated to be about 4·6%. In contrast, far lower prevalence rates have been observed in East Africans, American blacks, Indians (0·7%), and among die Chinese population (0·4%). While the causes of the disease are unknown, a genetic predisposition associated with environmental factors is assumed as a plausible aetiological explanation. The significance of the genetic background becomes evident with a concordance of approximately 60% in monozygotic twins. The disease has a strong association with HLA-C, with about two-thirds of patients carrying the HLA-Cw*0602 allele compared with only 10-15% in the general population. Carriers of this allele have a higher psoriasis risk and die disease becomes manifest at an earlier age in these patients. Psoriasis is considered as a T-cell mediated inflammatory skin disease. T-helper (Th) 1 lymphocytes produce autoreactive interferon (IFN)-γ and induce further cellular reactions, resulting in marked increases of keratinocyte proliferation, abnormal patterns of keratinocyte differentiation, concomitant inflammation, and dermal proliferation of small vessels.

There are distinct clinical phenotypes of the disease, including chronic plaque lesions (psoriasis vulgaris of type I, early onset and type II, late onset), guttate psoriasis, psoriatic erythroderma and drug-induced psoriasis as well as various forms of pustular psoriasis. At least 10% of patients develop arthritis. Apart from distinction of psoriasis types, the actual clinical status at the momentary stage of disease activity is an important factor determining the psoriatic phenotype.

**Atopic dermatitis** (AD) (a type of eczema) is an inflammatory, chronically relapsing, non-contagious and pruritic skin disease. Atopic dermatitis often occurs together with other atopic diseases like hay fever, asthma and conjunctivitis. It is a familial and chronic disease and its symptoms can increase or disappear over time. Atopic dermatitis in older children and adults is often confused with psoriasis. Atopic dermatitis afflicts humans, particularly young children; it is also a well-characterized disease in domestic dogs. Genomic research into the cause of multigenic diseases is still in its infancy: few genes have ever been identified that contribute to multigenic human disorders. Researchers have attempted to do this in past whole-genome screens for AE and related diseases, but their results have been inconsistent. A few of the pertinent loci have been validated by replication in further studies (chromosome 2q, chromosome 6p, and chromosome 12q, for example), but most have not been.

Other common types of acute and chronic dermatitis (eczema) are seborrheic dermatitis, nummular eczema, allergic contact dermatitis, hyperkeratotic rhagadiform eczema of hand and feet, dyshidrotic eczema (pompholyx), and other forms of dermatitis.

Although there are a number of therapeutics for these diseases, there is still a need for additional ways of treating these diseases.

The topical application of vitamin B12 has been used in the past for the treatment of atopic dermatitis as described in M. Stücker et al., British Journal of Dermatology 2004: 150: 977-983. Vitamin B12 is considered to act as a scavenger of nitric oxide. Patients were treated with a vitamin B12 containing preparation and the cream was considered superior to a placebo treatment. As NO is considered to be implicated in the pathogenesis of atopic eczema and psoriasis inflammatory cytokines stimulate the expression of inducible nitric oxide synthase (iNOS) in keratinocytes and other cell types.

Surprisingly and in contrast to expectations from the above-referenced document it has now been concluded that nitrosylcobalamin is a suitable agent for the treatment of skin diseases, especially inflammatory and hyperproliferative skin diseases selected from the group consisting of eczema, atopic dermatitis, psoriasis, acne and rosacea.

Nitrosylcobalamin is for example described in J. A. Bauer, Anti-Cancer Drugs 9 (1998) 239-244

Although nitrosylcobalamin is not able to act as a scavenger for NO, it has a positive effect in the treatment of atopic dermatitis and other types of eczema, psoriasis, acne and rosacea

The inventors of this invention found that nitrosylcobalamin is able to down-regulate inflammatory signaling, especially NF-κB-signalling, in epidermal keratinocytes and inflammatory cells of inflammatory and hyperproliferative skin diseases in human and veterinary medicine. According to this invention cobalamin is not used as a quenching system of free local NO but in contrast as a delivery system of NO to inflammatory and hyperproliferative cells expressing the ubiqitous plasma membrane transcobalamin II receptor (TC II-R). The receptor-mediated uptake of nitrosylcobalamin delivers NO to inflammatory and hyperproliferative cells of human and animal epidermis. The nitrosylcobalamin provides sufficient amounts of released NO to target cells in inflammatory skin diseases thereby inhibiting genes involved in the regulation of inflammation. One mechanism by which nitrosylcobalamin is believed to inhibit inflammation is its inhibition of IKK activation, characterized by decreased phosphorylation of IκBa and inhibition of NF-κB binding activity to promoter regions of genes regulating the expression of inflammatory cytokines and chemokines.

## Claims

1. Use of nitrosyl-cobalamin for the preparation of a medicament for the treatment of a inflammatory and hyperproliferative skin disease selected from the group consisting of atopic dermatitis, all kinds of eczema, psoriasis, acne and rosacea.

2. The use of claim 1, wherein the medicament is for topical treatment.

3. The use of claim 2, wherein the medicament is in the form of a cream, a gel, a lotion, a bath, a prepared dressing or other form of external application.

4. The use of claim 2 or 3, wherein the concentration of nitrosyl-cobalamin is 0.05 to 2% by weight.

5. A topical pharmaceutical preparation comprising nitrosyl-cobalamin.

6. The topical pharmaceutical preparation of claim 5 in the form of a cream, a gel, a lotion, a bath, a prepared dressing or other form of external application.

7. The topical pharmaceutical preparation of claim 5 wherein the concentration of nitrosyl-cobalamin is 0.05 to 2% by weight.

8. The topical pharmaceutical preparation of claim 5 wherein cobalamin is selected from NO-Cyanocobalamin, NO-Hydroxycobalamin and NO-Methylcobalamin.
